(19) Europäisches Patentamt
European Patent Office
Office européen des brevets

(11) **EP 4 338 762 A1**

(12) **EUROPEAN PATENT APPLICATION**


(43) Date of publication:
**20.03.2024 Bulletin 2024/12**

(21) Application number: **22195761.6**

(22) Date of filing: **15.09.2022**

(51) International Patent Classification (IPC):
***A61L 15/60*** ***(2006.01)*** ***A61L 15/24*** ***(2006.01)***
***A61L 15/26*** ***(2006.01)*** ***A61L 15/42*** ***(2006.01)***

(52) Cooperative Patent Classification (CPC):
(C-Sets available)
**A61L 15/60; A61L 15/24; A61L 15/26;**
**A61L 15/425** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Paul Hartmann AG**
**89522 Heidenheim (DE)**

(72) Inventors:
- **KETTEL, Markus**
  **89520 Heidenheim (DE)**
- **BASTIDA AGOTE, Nora**
  **89518 Heidenheim (DE)**

(74) Representative: **Paul Hartmann AG**
**Patents & Licensing**
**Paul-Hartmann-Straße 12**
**89522 Heidenheim (DE)**


(54) **PROCESS OF PRODUCING A HIGHLY ABSORBENT HYDROGEL COMPOSITION FOR MEDICAL PURPOSES, IN PARTICULAR FOR WOUND TREATMENT**


(57) The present invention relates to a process of producing a composition for medical purposes, in particular for wound treatment, comprising the steps of:
i. preparing a mixture comprising a superabsorbent polymer, an isocyanate-terminated prepolymer and water,
ii. allowing the prepolymer to react with one or more components of the mixture to obtain the composition,
wherein the superabsorbent polymer is a cross-linked polyacrylate and wherein the isocyanate-terminated prepolymer contains polyalkylene oxide units and is a branched molecule having at least three arms. Moreover, the present invention relates to the composition obtainable by the aforementioned process. Use of said composition in medicine and as wound dressing material is also a subject of the present invention. For example, the composition may be used as an atraumatic wound contact layer, which may absorb excess moisture from the wound and release moisture to the wound as required. Thereby, the composition may create a moist wound environment supporting the healing process and may effectively prevent maceration and drying of the wound. In particular, the composition may have a high absorption capacity.


Figure 1

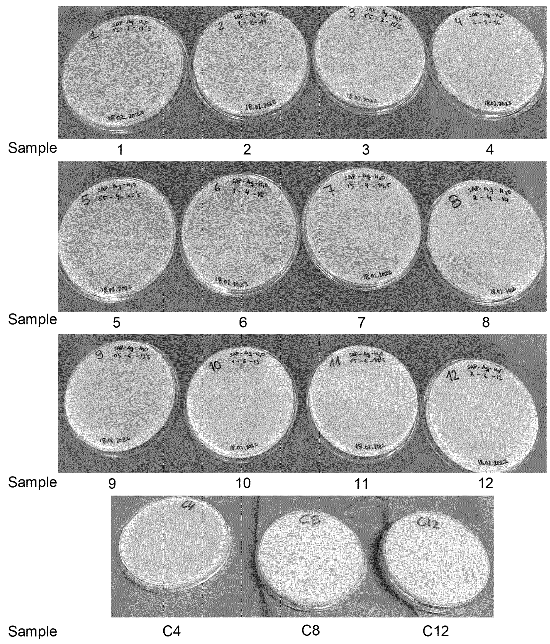


EP 4 338 762 A1

(52) Cooperative Patent Classification (CPC): (Cont.)

C-Sets
**A61L 15/24, C08L 33/08;**
**A61L 15/26, C08L 75/04**

## Description

The project leading to this application has received funding from the European Union's Horizon 2020 research and innovation programme under the Marie Sktodowska-Curie grant agreement No 955664.

The present invention relates to a process of producing a composition for medical purposes, in particular for wound treatment. Moreover, the present invention relates to the composition obtainable by the aforementioned process. Use of said composition in medicine and as wound dressing material is also a subject of the present invention.

WO 2010/000451 A1 discloses a multilayer wound dressing comprising a wound contact layer and an absorbent layer. The absorbent layer comprises a hydrophilic polyurethane foam having a water content of at least 10 % by weight. The wound contact layer may comprise a hydrogel. The wound dressing disclosed in WO'451 does not adhere to the wound and may create a moist wound environment beneficial for wound healing by allowing the wound dressing to both absorb excess moisture from the wound and release moisture to the wound as needed.

The wound dressing disclosed in WO'451 provide gentle and effective wound treatment in many cases. However, the capability of the wound dressing to absorb wound exudate may be insufficient when highly exuding wounds are treated.

The task of the present invention was to provide an improved composition for medical purposes, in particular for wound treatment. In particular, the composition should be suited for direct wound contact and provide a moist wound environment beneficial for wound healing, while at the same time being able to absorb higher amounts of wound exudate. These tasks are solved with a process according to claim 1 and a composition according to claim 16.

According to the invention, the process of producing a composition for medical purposes, in particular for wound treatment, comprises the following steps:

i. Preparing a mixture comprising a superabsorbent polymer, an isocyanate-terminated prepolymer and water,
ii. allowing the prepolymer to react with one or more components of the mixture to obtain the composition,

wherein the superabsorbent polymer is a cross-linked polyacrylate and
wherein the isocyanate-terminated prepolymer contains polyalkylene oxide units and is a branched molecule having at least three arms. Such branched prepolymers are sometimes also designated as star-shaped prepolymers (in the present case star-shaped, isocyanate-terminated prepolymer containing polyalkylene oxide units and at least three arms).

The inventors found that a cross-linked polyacrylate as superabsorbent polymer (SAP) and an at least three-armed branched isocyanate-terminated prepolymer containing polyalkylene oxide units may be used to produce a particular advantageous composition for medical purposes. Therefore, the composition produced by the process according to the invention is very well suited for medical purposes, in particular for wound treatment. The composition may be used as an atraumatic wound contact layer, which may absorb excess moisture from the wound and release moisture to the wound as required. Thereby, the composition may create a moist wound environment supporting the healing process and may effectively prevent maceration and drying of the wound. In particular, the composition may have a high absorption capacity. The superabsorbent polymer contained in the composition according to the invention is responsible for the high absorption capacity. The high absorption capacity of the selected superabsorbent polymer may also encompass binding of harmful components contained in the wound exudate such as bacteria, elastase and matrix metalloproteinases (MMPs).

When the process according to the invention is carried out, the prepolymer reacts by polyaddition with one or more components of the mixture to form the composition as final product. The prepolymer may react with the superabsorbent polymer. Specifically, the isocyanate group of the prepolymer may react with functional groups of the superabsorbent polymer, for example a carboxyl group, leading to the formation of an amide bond with the release of carbon dioxide. In addition, the prepolymer reacts with the water. In this case, the isocyanate group of the prepolymer is converted to an amine group with the release of carbon dioxide. Subsequently, this amine group then reacts with another isocyanate group of the prepolymer leading to the formation of a urea bond. Since the prepolymer usually contains urethane bonds, these functional groups are normally present in the composition as well. Therefore, the reactions described before typically result in the formation of a polyurea polymer, in particular a polyurethane-polyurea copolymer or a polyurethane-polyurea-polyamide polymer network. Therefore, the present composition typically comprises a polyurea polymer, in particular a polyurethane-polyurea copolymer or a polyurethane-polyurea-polyamide polymer.

In general, the present composition is a hydrogel. A hydrogel according to the present invention is essentially a disperse system with at least one solid hydrophilic phase and a liquid phase, namely water. The solid phase forms a sponge-like, three-dimensional cross-linked polymer network, which is filled by the water. The previously described reactions of the prepolymer form the solid cross-linked polymer network of the hydrogel. In conclusion, the present composition is typically a hydrogel consisting of a polyurea polymer, in particular a polyurethane-polyurea copolymer or a polyurethane-polyurea-polyamide polymer, and possibly the superabsorbent polymer (if the SAP does not or at least

not completely react with the prepolymer) as solid phase(s) and an aqueous liquid as liquid phase.

Since the prepolymer may react with the superabsorbent polymer, the superabsorbent polymer may be an integral part of the hydrogel's solid polymer network. The superabsorbent polymer may therefore be covalently bound within the composition. This may provide the composition with long-lasting, high absorbent properties as the superabsorbent polymer is fixed within the composition and cannot be released. However, the reaction of the superabsorbent polymer with the prepolymer is not mandatory for the present invention. A part or the complete amount of the superabsorbent polymer may only be embedded in the solid polymer network of the hydrogel composition being formed by the reaction of the prepolymer with the water.

As already explained, the present composition shall also provide moisture to the wound if required, for example in the case of a dry wound. Therefore, usually the present composition is not subjected to a drying step by the manufacturer.

The superabsorbent polymer may be a cross-linked sodium polyacrylate. Furthermore, the superabsorbent polymer may preferably be present in particulate form. That is, superabsorbent polymer (SAP) particles are preferably used to produce the present composition. Suitable SAP particles may be purchased from Evonik Nutrition & Care GmbH (Krefeld, Germany) under the trade name FAVOR® PAC 300. Typically, the superabsorbent polymer is used in essentially dry form in the present process. Therefore, it is intended that the superabsorbent polymer is essentially dry prior to preparing the mixture according to the invention. However, the superabsorbent polymer may contain small amounts of moisture, for example less than 6 %, preferably less than approximately 4 %, as determined according to Edana WSP 230.

In a preferred embodiment of the invention, the polyalkylene oxide units of the prepolymer are formed by polyethylene oxide and/or polypropylene oxide units, wherein the weight ratio of ethylene oxide to propylene oxide units is preferably 3 : 1 to 7 : 1. Preferably, the prepolymer has exactly three arms.

In particular, the isocyanate-terminated prepolymer is a three-armed copolymer of ethylene oxide and propylene oxide units, each of which has been terminally reacted with a molecule of isophorone diisocyanate. It usually has a content of reactive isocyanate end groups (NCO groups) of 2.5 % to 4.0 %, preferably 3.0 % to 3.4 %, particularly preferably 3.2 %, and a weight ratio of ethylene oxide units to propylene oxide units of 3 : 1 to 4 : 1. Such an isocyanate-terminated prepolymer with aliphatic isocyanate groups is commercially available, for example, as Aquapol® PI-13000-31 (Carpenter; Richmond, USA).

The figure below illustrates the schematic structure of a three-armed branched isocyanate-terminated prepolymer containing polyethylene oxide and polypropylene oxide units (as in Aquapol®). A glycerol molecule forms the center of the prepolymer. The three "arms" of the prepolymer, each with a terminal isocyanate group, are linked to the hydroxyl groups of the glycerol molecule. The glycerol molecule itself is not shown in the figure. It would be located in the right half of the picture where the three "arms" schematized as a wavy line meet. In the left half of the picture, the chemical structure of an "arm" is shown in more detail.

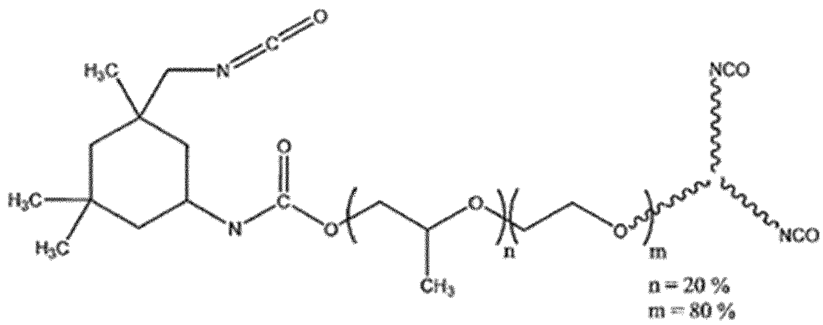

n = 20 %
m = 80 %

In a simple and cost-efficient embodiment of the invention, the mixture consists of the superabsorbent polymer, the prepolymer and the water. That is, for producing the composition only the superabsorbent polymer, the prepolymer and the water are used. Alternatively, the mixture may comprise further components in addition to the superabsorbent polymer, the prepolymer and the water. For instance, the mixture may further comprise a humectant. Preferably, the humectant is ethylene glycol, propylene glycol, PEG300, PEG2000, glycerol, sucrose or sorbitol. In particular, the humectant is glycerol. The mixture may further comprise an inorganic salt. Preferably, the salt is sodium chloride, potassium chloride, magnesium chloride, calcium chloride or a mixture of at least two of these salts. In particular, the salt is sodium chloride. On the other hand, the presence of a surfactant in the mixture is less preferred. Consequently, the mixture preferably does not contain a surfactant. Similarly, the presence of a foaming agent in the mixture is less preferred. Consequently, the mixture preferably does not contain a foaming agent. In this regard, the superabsorbent polymer and the water are not considered as foaming agents, even though both components may have some foaming activity by the release of carbon dioxide if they react with the prepolymer. By omitting surfactants and foaming agents the production of the composition may be simpler, less expensive and more environmentally friendly. The biocompatibility of the composition may also increase if surfactants are avoided.

Preferably, the following amounts of superabsorbent polymer, prepolymer, water and optional further components such as the aforementioned humectant and inorganic salt may be used to produce the composition:

- Preferably, the proportion of the superabsorbent polymer, based on the total weight of the mixture, is 3 to 10 % by weight.
- Preferably, the proportion of the prepolymer, based on the total weight of the mixture, is 10 to 30 % by weight.
- Preferably, the combined proportion of the superabsorbent polymer and the prepolymer, based on the total weight of the mixture, is 10 to 50 % by weight, in particular 13 to 40 % by weight.
- Preferably, the proportion of the water, based on the total weight of the mixture, is 50 to 90 % by weight, in particular 60 to 87 % by weight.
- Preferably, the proportion of one or more further components present in the mixture, based on the total weight of the mixture, is 0 to 30 % by weight, in particular 0 to 20 % by weight. The specified ranges refer to the sum of all further components present in the mixture.
- Preferably, the proportion of the humectant, based on the total weight of the mixture, is 0 to 25 % by weight, in particular 0 to 7 % by weight. If a humectant is contained in the mixture, preferably at least 5 % by weight of the humectant are used, based on the total weight of the mixture.
- Preferably, the proportion of the inorganic salt, based on the total weight of the mixture, is 0 to 5 % by weight, in particular 0 to 1.5 % by weight. If an inorganic salt is contained in the mixture, preferably at least 0.5 % by weight of the inorganic salt are used, based on the total weight of the mixture.

The proportion of the superabsorbent polymer, the proportion of the prepolymer, the proportion of the water and the proportion of the further components add up to 100 % by weight. If no further components are included in the mixture, the proportion of the superabsorbent polymer, the proportion of the prepolymer and the proportion of the water add up to 100 % by weight.

As already pointed out, the compositions proposed here are generally hydrogels. However, depending on the proportions of the superabsorbent polymer, the prepolymer and the water in the mixture, the external appearance of the compositions may differ. Thus, in terms of the external appearance, the composition may be present as a gel, as a gel foam, or as a foam.

When the composition is present as a gel, it contains no or few voids and cells, respectively. The composition may then form a continuous, discrete layer. The composition may also be transparent or at least partial transparent when present as a gel. Thus, when the composition is present as a gel, it has an external appearance common to hydrogels.

When the composition is present as a foam, it is porous and contains a plurality of open and/or closed voids and cells, respectively. The foam composition is typically white and opaque, as well as more voluminous compared to a gel composition of the same mass. In addition, the foam composition contains a higher solid polymeric content and less water compared to a gel composition of the same mass.

The variant of the composition designated as "gel foam" is a transitional form between gel and foam. The "gel foam" can also be understood as a pre-swollen hydrophilic foam.

In the following, different embodiments of the invention are proposed, in which the previously mentioned ranges are further specified. The compositions according to these embodiments differ in consistency and are present either as a gel, gel foam or foam.

To obtain a composition of the gel type, the following amounts of superabsorbent polymer, prepolymer, water and optional further components may be selected:

- Preferably, the proportion of the superabsorbent polymer, based on the total weight of the mixture, is 3 to 10 % by weight.
- Preferably, the proportion of the prepolymer, based on the total weight of the mixture, is approximately 10 % by weight.
- Preferably, the combined proportion of the superabsorbent polymer and the prepolymer, based on the total weight of the mixture, is 13 to 20 % by weight.
- Preferably, the proportion of the water, based on the total weight of the mixture, is 80 to 87 % by weight.
- Preferably, the proportion of one or more further components present in the mixture, based on the total weight of the mixture, is 0 to 7 % by weight. The specified range refers to the sum of all further components present in the mixture.

Again, the proportion of the superabsorbent polymer, the proportion of the prepolymer, the proportion of the water and the proportion of the further components add up to 100 % by weight. If no further components are included in the mixture, the proportion of the superabsorbent polymer, the proportion of the prepolymer and the proportion of the water add up to 100 % by weight.

The gel-like composition may have a high water-releasing capacity and is thus particularly suitable for the treatment of dry wounds.

To obtain a composition of the gel foam type, the following amounts of superabsorbent polymer, prepolymer, water and optional further components may be selected:

- Preferably, the proportion of the superabsorbent polymer, based on the total weight of the mixture, is 3 to 10 % by weight.
- Preferably, the proportion of the prepolymer, based on the total weight of the mixture, is approximately 20 % by weight.
- Preferably, the combined proportion of the superabsorbent polymer and the prepolymer, based on the total weight of the mixture, is 23 to 30 % by weight.
- Preferably, the proportion of the water, based on the total weight of the mixture, is 70 to 77 % by weight.
- Preferably, the proportion of one or more further components present in the mixture, based on the total weight of the mixture, is 0 to 7 % by weight. The specified range refers to the sum of all further components present in the mixture.

Again, the proportion of the superabsorbent polymer, the proportion of the prepolymer, the proportion of the water and the proportion of the further components add up to 100 % by weight. If no further components are included in the mixture, the proportion of the superabsorbent polymer, the proportion of the prepolymer and the proportion of the water add up to 100 % by weight.

The gel foam may both release a comparatively large amount of water and absorb a comparatively large amount of water, making it suitable for treating a wide variety of wound types.

To obtain a composition of the foam type, the following amounts of superabsorbent polymer, prepolymer, water and optional further components may be selected:

- Preferably, the proportion of the superabsorbent polymer, based on the total weight of the mixture, is 3 to 10 % by weight.
- Preferably, the proportion of the prepolymer, based on the total weight of the mixture, is approximately 30 % by weight.
- Preferably, the combined proportion of the superabsorbent polymer and the prepolymer, based on the total weight of the mixture, is 33 to 40 % by weight.
- Preferably, the proportion of the water, based on the total weight of the mixture, is 60 to 67 % by weight.
- Preferably, the proportion of one or more further components present in the mixture, based on the total weight of the mixture, is 0 to 7 % by weight. The specified range refers to the sum of all further components present in the mixture.

Again, the proportion of the superabsorbent polymer, the proportion of the prepolymer, the proportion of the water and the proportion of the further components add up to 100 % by weight. If no further components are included in the mixture, the proportion of the superabsorbent polymer, the proportion of the prepolymer and the proportion of the water add up to 100 % by weight.

The foam-like composition may have a particular high water absorption capacity and is thus very well suited for the treatment of heavily exuding wounds.

It is particularly preferred that the composition has an absorption capacity of at least 10 g/g, preferably at least 20 g/g, in particular at least 30 g/g. However, the absorption capacity of the composition may be limited to 45 g/g. Therefore, the composition may have an absorption capacity of 10 to 45 g/g, preferably 20 to 45 g/g, in particular 30 to 45 g/g. The absorption capacity may be determined with the test method described in the present specification. To achieve such an absorption capacity the mixture may comprise at least 3 % by weight, preferably at least 5 % by weight, more preferably at least 8 % by weight and in particular approximately 10 % by weight superabsorbent polymer, based on the total weight of the mixture. For example, the mixture may comprise 3 to 10 % by weight, preferably 5 to 10 % by weight, in particular 8 to 10 % by weight superabsorbent polymer, based on the total weight of the mixture.

According to another advantageous embodiment of the invention the composition has a moisture donation capacity of at least 6 $mg/cm^2$, in particular at least 10 $mg/cm^2$. The moisture donation capacity of the composition may nevertheless be limited to 20 $mg/cm^2$. Therefore, the composition may have a moisture donation capacity of 6 to 20 $mg/cm^2$, in particular 10 to 20 $mg/cm^2$. The moisture donation capacity may be determined with the test method described in the present specification.

As mentioned in the beginning, the present invention also relates to the composition obtainable by the process previously described. Subject of the present invention is also the use of said composition in medicine, in particular in wound treatment (first and second medical indication). Accordingly, said composition for use in medicine, in particular for use in wound treatment, is claimed. In particular, the composition may be used to treat heavily exuding wounds due to its excellent absorption capacity.

When the composition is used in wound treatment, it is typically incorporated into a wound dressing. Accordingly, claim 18 herein also claims a wound dressing comprising the composition in any of its embodiments. Such a wound

dressing may, for example, comprise a backing layer and a wound contact layer, with the composition forming the wound contact layer. Then, the composition can best exert its beneficial properties such as absorbing wound exudate or releasing moisture to the wound. The backing layer may be a water impermeable, water-vapor permeable film layer made of, for instance, polyurethane. To fix the backing layer to the wound contact layer, the dressing may advantageously further comprise an adhesive such as an acrylic glue between the backing and the wound contact layer. Even though it would be possible to include further absorbent layers into the dressing, this will not be necessary in many cases due to the excellent absorption capacity of the present composition. Therefore, in a preferred embodiment of the invention the dressing does not comprise further absorbent layers apart from the wound contact layer. This may reduce the structural complexity of the dressing, allowing a simplified production and cost savings.

**Examples and Figures**

The examples and figures described below are intended to explain the invention in more detail. However, the described examples and figures shall not limit the scope of the present invention in any way.

1. Preparation of exemplary compositions

Briefly, the samples were prepared as follows. Solid SAP particles were placed in a petri dish. Then, an aqueous solution consisting of prepolymer and demineralized water was added to the petri dish using a laboratory gel casting machine (B100 from bdtronic). Subsequently, the petri dish was gently agitated to mix the SAP particles with the aqueous prepolymer solution. The resulting mixture polymerized automatically, yielding a gel-like, gelfoam-like or foam-like sample, depending on the amounts of SAP, prepolymer and water used. To protect the sample during storage, the petri dish was sealed with parafilm.

All samples were prepared with FAVOR® PAC 300 SAP particles (Evonik Nutrition & Care GmbH, Krefeld, Germany) and Aquapol® PI-13000-31 star-shaped isocyanate-terminated prepolymer (Carpenter; Richmond, USA).

**Table 1** summarizes the prepared samples and their properties including the control samples C4, C8 and C12. The control samples did not contain SAP (and are thus no compositions according to the invention).

Table 1: Prepared samples and their properties

| Sample No. | SAP [weight %] | Prepolymer [weight %] | Water [weight %] | SAP + Prepolymer [weight %] | consistency |
|---|---|---|---|---|---|
| 1 | 3 | 10 | 87 | 13 | gel |
| 2 | 5 | 10 | 85 | 15 | gel |
| 3 | 8 | 10 | 82 | 18 | gel |
| 4 | 10 | 10 | 80 | 20 | gel |
| C4 | 0 | 20 | 80 | 20 | gel |
| 5 | 3 | 20 | 77 | 23 | gel foam |
| 6 | 5 | 20 | 75 | 25 | gel foam |
| 7 | 8 | 20 | 72 | 28 | gel foam |
| 8 | 10 | 20 | 70 | 30 | gel foam |
| C8 | 0 | 30 | 70 | 30 | gel foam |
| 9 | 3 | 30 | 67 | 33 | foam |
| 10 | 5 | 30 | 65 | 35 | foam |
| 11 | 8 | 30 | 62 | 38 | foam |
| 12 | 10 | 30 | 60 | 40 | foam |
| C12 | 0 | 40 | 60 | 40 | foam |

**Figure 1** shows the prepared samples. All samples are colored white, but to varying degrees. As can be seen in the photographs, the gel-like as well as the gel foam-like samples may be at least partial transparent. In contrast, the foam-like samples are opaque.

2. Absorption capacity of the exemplary compositions

The samples from Table 1 were tested with regard to their absorption capacity. To measure the absorption capacity, sample pieces with a size of 1 cm x 1 cm were punched out and weighed. They were then placed in a beaker containing demineralized water for 24 hours. They were then removed from the beaker and weighed again. Each sample was tested three times. The absorption capacity is calculated using the following equation and is expressed in units of g/g:

$$\text{Absorption capacity} = (\text{final weight} - \text{initial weight}) / \text{initial weight}$$

**Figures 2 to 4** show the results of the absorption capacity tests. **Figure 2** refers to the gel-like samples 1 to C4, **figure 3** refers the gel foam-like samples 5 to C8 and **figure 4** refers to the foam-like samples 9 to C12. The absorption capacity of the samples mainly depends on their SAP content. Therefore, even the gel-like samples may absorb large quantities of water. In addition, the absorption capacity depends on the consistency of the samples. In particular, the foam-like samples may absorb more liquid than the gel-like samples with the same SAP amount.

3. Moisture donation capacity of the exemplary compositions

The samples from Table 1 were tested for their moisture donation capacity. This test was used to determine how much moisture the samples can release to filter papers. This can be an indication of how much moisture the compositions in a wound dressing can release to the wound surface.

The test procedure was as follows:
Round sample pieces with a diameter of 5 cm were first punched from the prepared samples. Each sample was placed in a petri dish lined with five filter papers, the weight of which was determined beforehand (initial weight). The diameter of the petri dish and filter paper was 5 cm. The petri dishes were closed with lids and additionally sealed with parafilm to prevent moisture loss.

Subsequently, the samples were left in the sealed petri dishes at room temperature for a period of 24 hours.

Then, the petri dishes were opened and weighed again without the samples (final weight) to calculate the moisture donation capacity in mg/cm$^2$. Each sample was analyzed once.

$$\text{Moisture donation capacity} = ((\text{final weight} - \text{initial weight}) \times 1000) / A$$

Initial weight: weight of the petri dish and filter papers at the start of the test ("dry") in g Final weight: weight of the petri dish and the filter papers after the test ("moistened") in g A: Area of the sample in cm$^2$ (approximately 19.6 cm$^2$ in the present case)

**Figures 5 to 7** show the results of the moisture donation capacity tests. **Figure 5** refers to the gel-like samples 1 to C4, **figure 6** refers the gel foam-like samples 5 to C8 and **figure 7** refers to the foam-like samples 9 to C12. All samples showed favorable moisture donation capacities. The gel-like samples possess the highest moisture donation capacity, making them particularly suited for treating dry wounds.

4. Wound dressing with the composition

**Figure 8** shows an exemplary island wound dressing with a composition according to the invention in schematic form. The wound dressing comprises a backing layer **1,** which is provided with an adhesive layer **2** over its entire surface. The backing layer **1** can be, for example, a water impermeable, water-vapor permeable polyurethane film which is coated over its entire surface with an acrylate adhesive as adhesive layer **2.** The backing layer **1** serves as a load-bearing and covering layer in the wound dressing. Amongst other things, the adhesive layer **2** enables the wound dressing to be attached to the patient's body. Furthermore, the wound dressing comprises a wound contact layer **3,** which is formed by the composition according to the invention. The wound contact layer **3** is bonded to the backing layer **1** via the adhesive layer **2.** When the wound dressing is attached to the patient's body, the wound contact layer **3** is placed directly on the wound. With the wound contact layer **3,** the wound dressing can absorb wound exudate and release moisture to the wound.

It is also conceivable for the wound dressing to comprise further layers. For example, a further absorbent layer can be arranged between the adhesive layer **2** and the wound contact layer **3** in order to increase the absorption capacity of the wound dressing. In this regard, the absorbent layer may comprise, for example, a hydrophilic polyurethane foam or a nonwoven fabric. However, as already mentioned such an additional absorbent layer is usually not required due to the excellent absorbent properties of the hydrogel composition forming the wound contact layer **3.**

The wound dressing can be produced by directly casting the composition in a liquid state (i.e., the reaction mixture) onto the backing layer **1** and its adhesive layer **2,** respectively. The finished wound dressing is then available after the composition has cured. Alternatively, the wound contact layer **3** can also be produced in a separate casting mold and then bonded to the backing layer **1** via its adhesive layer **2.**

## Claims

1. Process of producing a composition for medical purposes, in particular for wound treatment, comprising the steps of:

    i. preparing a mixture comprising a superabsorbent polymer, an isocyanate-terminated prepolymer and water,
    ii. allowing the prepolymer to react with one or more components of the mixture to obtain the composition,

    wherein the superabsorbent polymer is a cross-linked polyacrylate and
    wherein the isocyanate-terminated prepolymer contains polyalkylene oxide units and is a branched molecule having at least three arms.

2. Process according to claim 1, wherein the composition comprises a polyurea polymer, in particular a polyurethane-polyurea copolymer or a polyurethane-polyurea-polyamide polymer.

3. Process according to claim 1 or 2, wherein the composition is not subjected to a drying step by the manufacturer.

4. Process according to anyone of the preceding claims, wherein the superabsorbent polymer is present in particulate form.

5. Process according to anyone of the preceding claims, wherein the polyalkylene oxide units are formed by polyethylene oxide and/or polypropylene oxide units, wherein the weight ratio of ethylene oxide to propylene oxide units is preferably 3 : 1 to 7 : 1.

6. Process according to anyone of the preceding claims, wherein the prepolymer has exactly three arms.

7. Process according to anyone of the preceding claims, wherein the prepolymer is obtained by a terminal reaction with a molecule of isophorone diisocyanate.

8. Process according to anyone of the preceding claims, wherein the mixture consists of superabsorbent polymer, prepolymer and water.

9. Process according to anyone of the preceding claims, wherein

    - the combined proportion of the superabsorbent polymer and the prepolymer, based on the total weight of the mixture, is 10 to 50 % by weight, in particular 13 to 40 % by weight,
    - the proportion of the water, based on the total weight of the mixture, is 50 to 90 % by weight, in particular 60 to 87 % by weight, and
    - the proportion of one or more further components present in the mixture, based on the total weight of the mixture, is 0 to 30 % by weight, in particular 0 to 20 % by weight.

10. Process according to anyone of the preceding claims, wherein

    - the combined proportion of the superabsorbent polymer and the prepolymer, based on the total weight of the mixture, is 13 to 20 % by weight,
    - the proportion of the water, based on the total weight of the mixture, is 80 to 87 % by weight, and
    - the proportion of one or more further components present in the mixture, based on the total weight of the mixture, is 0 to 7 % by weight.

11. Process according to anyone of the claims 1 to 9, wherein

    - the combined proportion of the superabsorbent polymer and the prepolymer, based on the total weight of the mixture, is 23 to 30 % by weight,

- the proportion of the water, based on the total weight of the mixture, is 70 to 77 % by weight, and
- the proportion of one or more further components present in the mixture, based on the total weight of the mixture, is 0 to 7 % by weight.

**12.** Process according to anyone of the claims 1 to 9, wherein

- the combined proportion of the superabsorbent polymer and the prepolymer, based on the total weight of the mixture, is 33 to 40 % by weight,
- the proportion of the water, based on the total weight of the mixture, is 60 to 67 % by weight, and
- the proportion of one or more further components present in the mixture, based on the total weight of the mixture, is 0 to 7 % by weight.

**13.** Process according to anyone of the preceding claims, wherein the composition has an absorption capacity of at least 10 g/g, preferably at least 20 g/g, in particular at least 30 g/g.

**14.** Process according to anyone of the preceding claims, wherein the mixture comprises at least 3 % by weight, preferably at least 5 % by weight, more preferably at least 8 % by weight and in particular approximately 10 % by weight superabsorbent polymer, based on the total weight of the mixture.

**15.** Process according to anyone of the preceding claims, wherein the composition has a moisture donation capacity of at least 6 $mg/cm^2$, in particular at least 10 $mg/cm^2$.

**16.** Composition, obtainable by a process according to anyone of the preceding claims.

**17.** Composition according to claim 16 for use in medicine, in particular for use in wound treatment.

**18.** Wound dressing, comprising a composition according to claim 16.

**19.** Wound dressing according to claim 18, wherein the wound dressing comprises a backing layer and a wound contact layer, wherein the composition forms the wound contact layer.

**20.** Wound dressing according to claim 19, wherein the dressing does not comprise further absorbent layers apart from the wound contact layer.

Figure 1

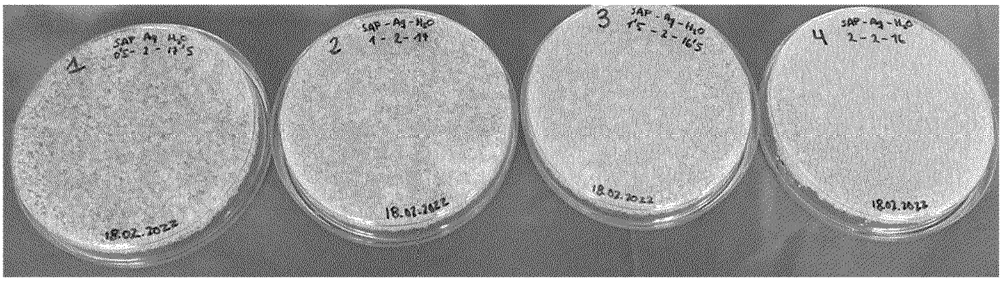

Sample 1 2 3 4

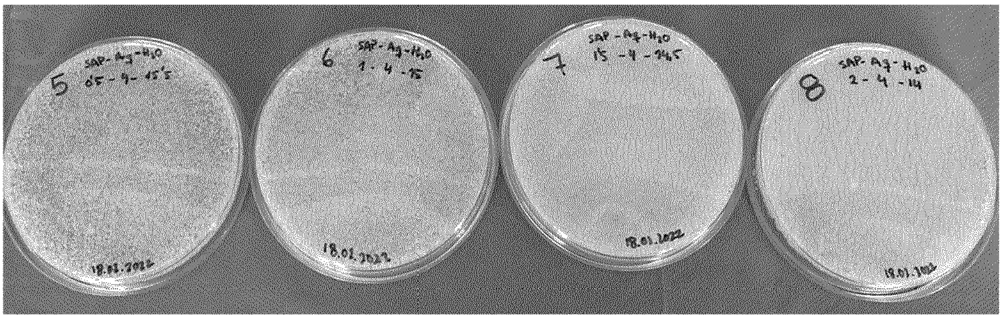

Sample 5 6 7 8

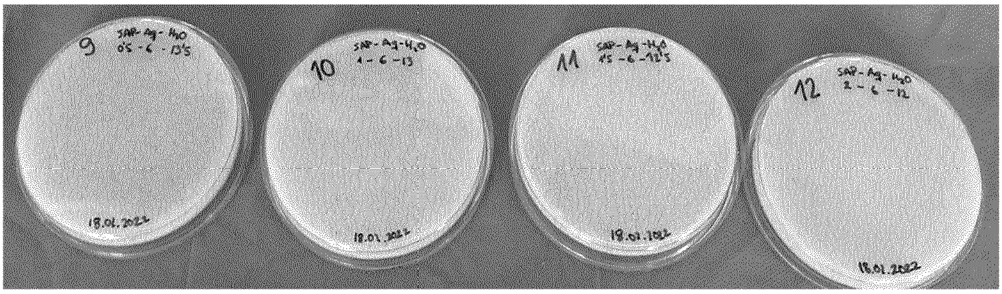

Sample 9 10 11 12

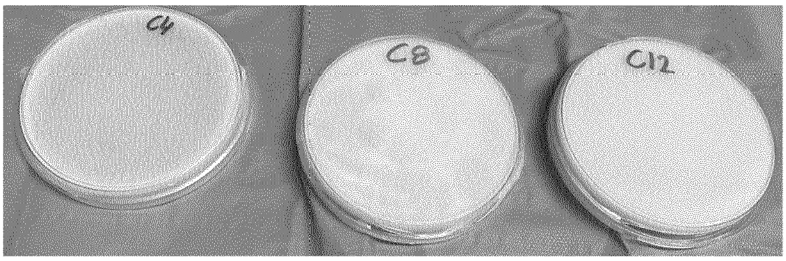

Sample C4 C8 C12

Figure 2

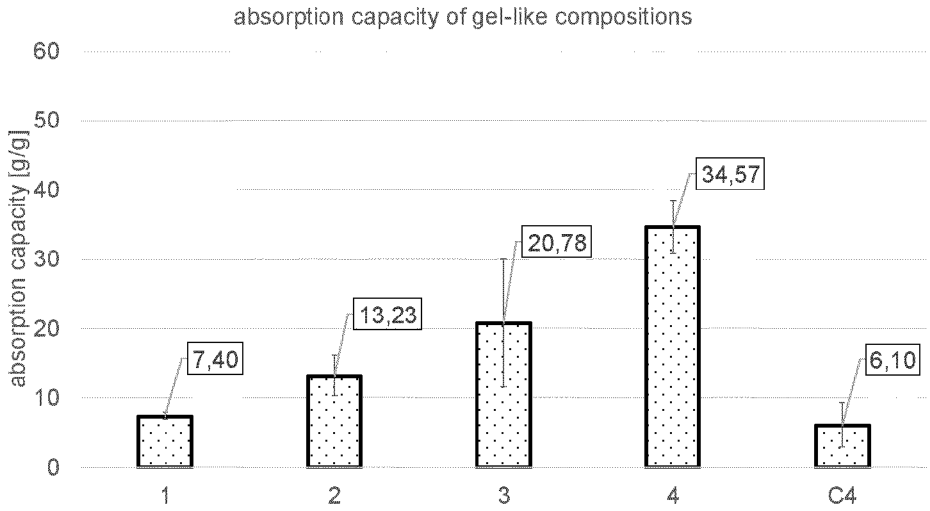


Figure 3

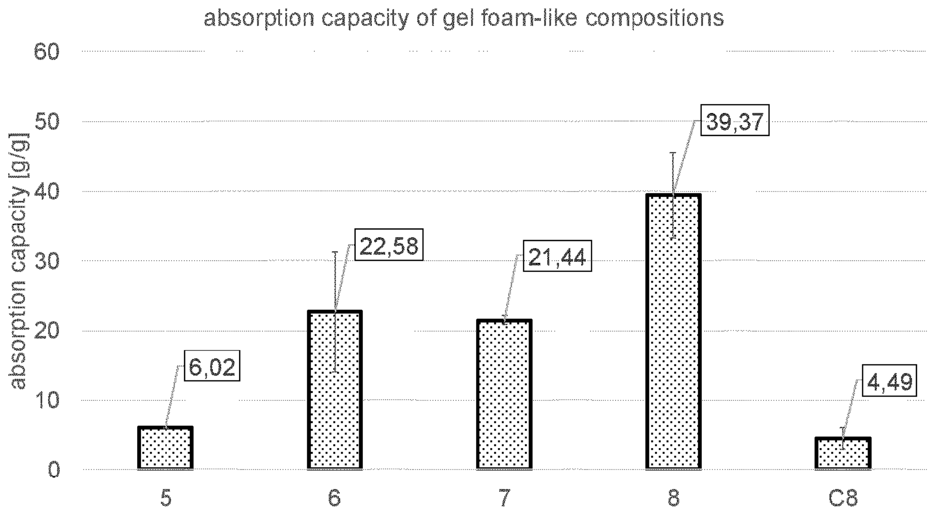


Figure 4

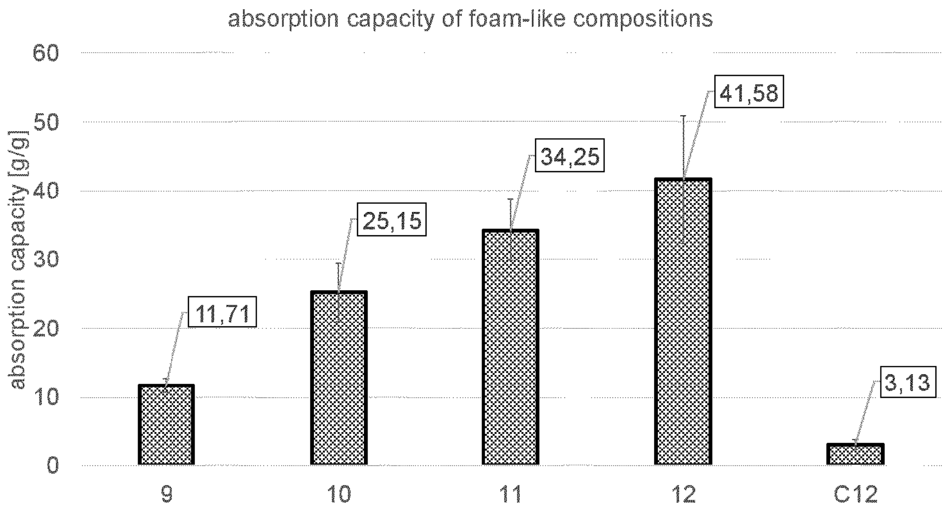

Figure 5

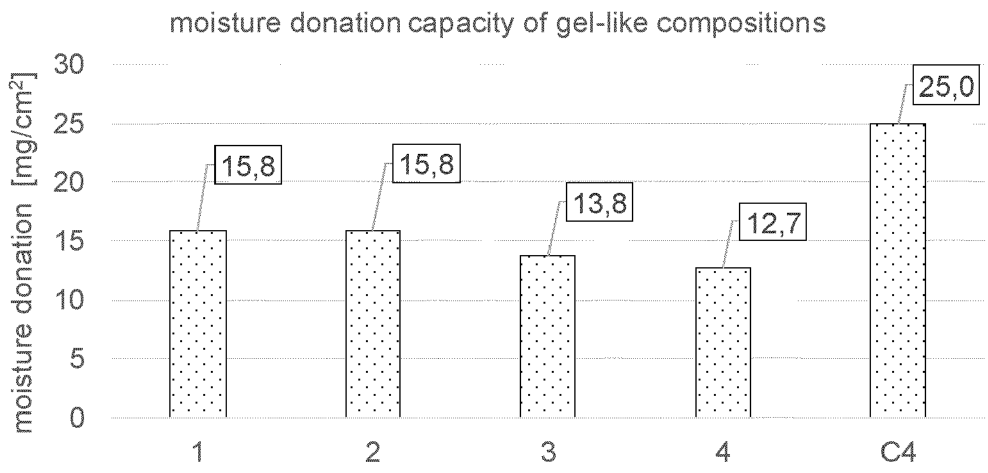


Figure 6

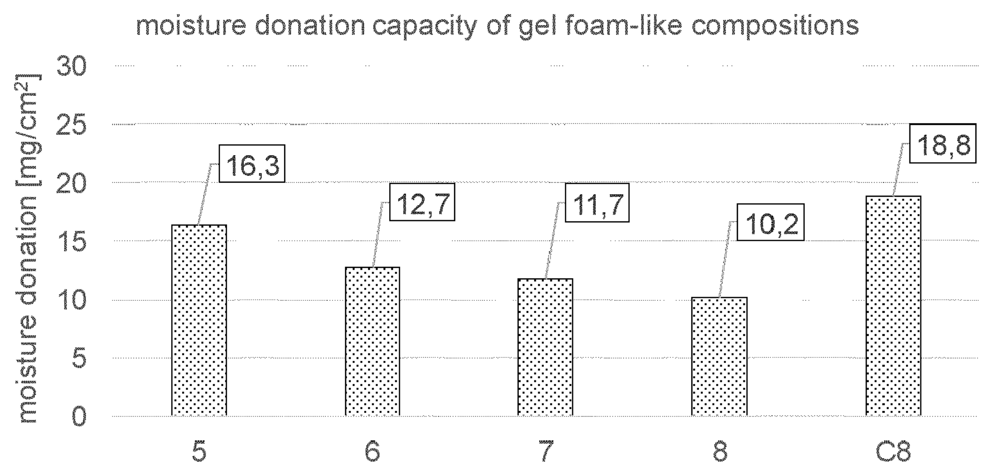


Figure 7

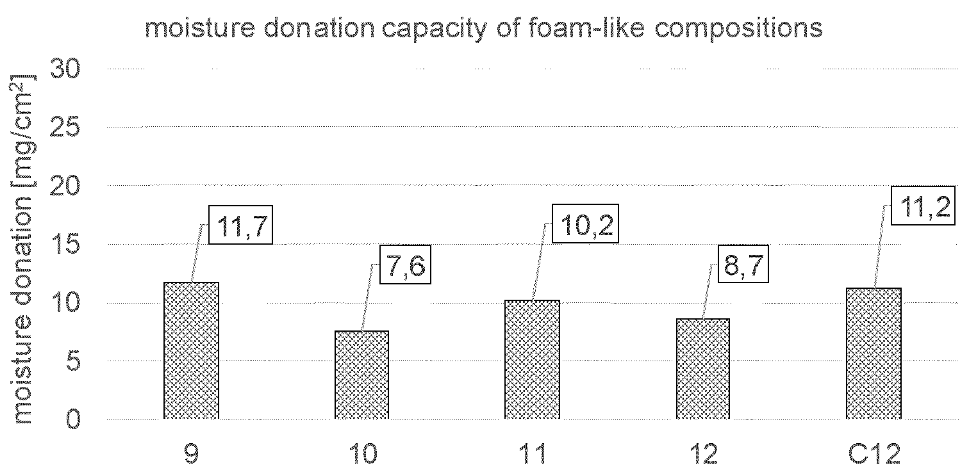

Figure 8

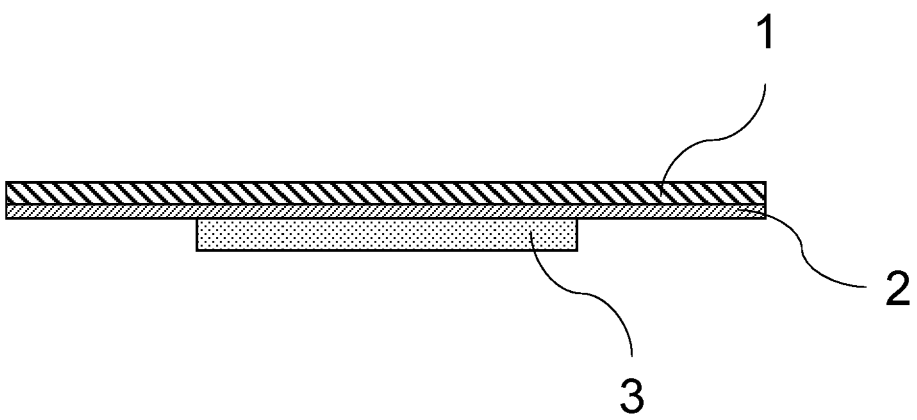

Europäisches Patentamt
European Patent Office
Office européen des brevets

# EUROPEAN SEARCH REPORT

Application Number
EP 22 19 5761

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 793 681 A1 (ICI PLC [GB]) 10 September 1997 (1997-09-10)<br>* paragraph [0008] *<br>* paragraph [0028] - paragraph [0030] *<br>* paragraph [0044] - paragraph [0046] *<br>* paragraph [0048] - paragraph [0049] *<br>* paragraph [0053] *<br>* paragraph [0055] *<br>* examples 1,3 *<br>* table 5 *<br>----- | 1-18 | INV.<br>A61L15/60<br>A61L15/24<br>A61L15/26<br>A61L15/42 |
| X | EP 3 235 520 A1 (COVESTRO DEUTSCHLAND AG [DE]) 25 October 2017 (2017-10-25)<br>* paragraph [0001] *<br>* paragraph [0013] *<br>* paragraph [0015] *<br>* paragraph [0017] - paragraph [0018] *<br>* paragraph [0023] *<br>* paragraph [0027] *<br>* paragraph [0035] - paragraph [0037] *<br>* paragraph [0083] *<br>* paragraph [0087] *<br>* example 2 *<br>----- | 1,2,4-7,<br>13-20 | |

TECHNICAL FIELDS SEARCHED (IPC)

A61L

The present search report has been drawn up for all claims

1

| Place of search | Date of completion of the search | Examiner |
|---|---|---|
| The Hague | 23 February 2023 | Zalfen, Alina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 19 5761

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2023

| Patent document cited in search report | Publication date | Patent family member(s) | Publication date |
|---|---|---|---|
| EP 0793681 A1 | 10-09-1997 | AR 000912 A1 | 27-08-1997 |
| | | AR 006923 A2 | 29-09-1999 |
| | | AR 010462 A2 | 28-06-2000 |
| | | AR 010463 A2 | 28-06-2000 |
| | | AT 182341 T | 15-08-1999 |
| | | AU 701887 B2 | 11-02-1999 |
| | | BG 63344 B1 | 31-10-2001 |
| | | BR 9509743 A | 21-10-1997 |
| | | CA 2203516 A1 | 30-05-1996 |
| | | CN 1164243 A | 05-11-1997 |
| | | CN 1439660 A | 03-09-2003 |
| | | CZ 287677 B6 | 17-01-2001 |
| | | DE 69510953 T2 | 09-12-1999 |
| | | DK 0793681 T3 | 24-01-2000 |
| | | EP 0793681 A1 | 10-09-1997 |
| | | EP 0894814 A1 | 03-02-1999 |
| | | ES 2135774 T3 | 01-11-1999 |
| | | FI 972171 A | 21-05-1997 |
| | | GR 3031072 T3 | 31-12-1999 |
| | | HK 1002660 A1 | 11-09-1998 |
| | | HU 216283 B | 28-06-1999 |
| | | JP 3761575 B2 | 29-03-2006 |
| | | JP H10509473 A | 14-09-1998 |
| | | JP 2005113155 A | 28-04-2005 |
| | | KR 100359041 B1 | 17-02-2003 |
| | | MY 113411 A | 28-02-2002 |
| | | NO 312517 B1 | 21-05-2002 |
| | | PL 320365 A1 | 29-09-1997 |
| | | RO 120139 B1 | 30-09-2005 |
| | | SI 9520123 A | 31-12-1997 |
| | | TR 199501467 A2 | 21-07-1996 |
| | | WO 9616099 A1 | 30-05-1996 |
| EP 3235520 A1 | 25-10-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010000451 A1